# EUROPÄISCHE PATENTSCHRIFT

(11) **EP 0 865 265 B1**
(45) Veröffentlichungstag und Bekanntmachung des Hinweises auf die Patenterteilung: **21.04.1999**
(21) Anmeldenummer: 96943894.4
(22) Anmeldetag: 06.12.1996
(51) Int. Cl.: A61F 17/00

(54) **EINRICHTUNG ZUM TEMPERIEREN VON HAUTPARTIEN DES MENSCHLICHEN KÖRPERS**
DEVICE FOR REGULATING THE TEMPERATURE OF SKIN PARTS OF THE HUMAN BODY
DISPOSITIF POUR REGULER LA TEMPERATURE DE PARTIES DE CUTANEES DU CORPS HUMAIN

(30) Priorität: 08.12.1995 DE 19545792; 10.05.1996 DE 19618787; 28.06.1996 DE 19625995
(43) Veröffentlichungstag der Anmeldung: 23.09.1998
(73) Patentinhaber: Schmidt & Lenhardt oHG, 71720 Oberstenfeld (DE)
(72) Erfinder: SCHMIDT, Peter, D-88260 Argenbühl (DE)
(74) Vertreter: Hübner, Hans-Joachim, Dipl.-Ing.
(86) Internationale Anmeldenummer: EP9605466
(87) Internationale Veröffentlichungsnummer: WO9721412

(56) Entgegenhaltungen:
- EP-A- 0 039 443
- WO-A-95/10251
- DE-A- 3 505 274
- US-A- 3 606 890
- US-A- 5 411 541

## Beschreibung

Die Erfindung betrifft eine Einrichtung zum Temperieren von Hautpartien des menschlichen Körpers, mit einem am Körper anlegbaren Kissen, das ein Flüssigkeitskanalsystem mit einer Einlaß- und einer Auslaßöffnung aufweist, mit einer, an die Einlaßöffnung angeschlossenen Zulaufleitung und einer, an die Auslaßöffnung angeschlossenen Rücklaufleitung, weiterhin mit einem Versorgungsgerät, das einen wärmeisolierten Behälter mit Deckel sowie eine Pumpe umfaßt, die saugseitig mit dem Inneren des Behälters verbunden ist, und mit einem, an die Pumpe angeschlossenen Versorgungsanschluß, mit der die Zulaufleitung kuppelbar ist und mit einer Ausbildung, die die Rücklaufleitung mit dem Behälter verbindet.

Einrichtungen dieser Art sind aus der WO 95/10251, EP-0039443, US-5,411,541, US-3,807,939, US-5,190,033, DE-3505274 bekannt. Nachteilig ist bei den bekannten Vorschlägen, daß an das Versorgungsgerät nur ein einziges Therapiekissen angeschlossen werden kann. In der Praxis besteht aber ein dringendes Bedürfnis, bei Patienten gleichzeitig mehrere distanzierte Hautoberflächen mit Wärme und/oder Kälte zu behandeln. Nach dem Stand der Technik würde jedes Kissen ein eigenes Versorgungsgerät benötigen. Eine andere Möglichkeit bestände darin, eine gemeinsame Zulaufleitung und eine gemeinsame Rücklaufleitung für mehrere Kissen vorzusehen und jeweils T-Abzweigungen einzubauen. Dies hätte aber wiederum den Nachteil, daß die einzelnen Kissen nicht individuell beaufschlagt werden können.

Aufgabe der Erfindung ist es, eine Temperiereinrichtung der eingangs genannten Art zu schaffen, mit der gleichzeitig mehrere Therapiekissen von einem Versorgungsgerät mit temperierter Flüssigkeit beschickt werden können und dennoch eine individuelle Beaufschlagung der Kissen mit temperierter Flüssigkeit möglich ist.

Diese Aufgabe wird mit den Merkmalen des Patentanspruches 1 gelöst.

Bevorzugte Ausgestaltungen der Erfindung sind in den abhängigen Ausprüchen angegeben.

Anhand der Zeichnung wird die Erfindung beispielsweise näher beschrieben.

Es zeigt:
- FIG. 1: eine Ansicht eines Versorgungsgerätes mit mehreren gleichzeitig angeschlossenen Kissen, die über separate Zulauf- und Rücklaufleitungen mit dem Versorgungsgerät verbunden sind.
- FIG. 2: eine Ansicht mehrerer kleinerer Kissen am Kopf eines Patienten, bei denen die Zulaufleitungen koaxial in den Rücklaufleitungen geführt sind, sodaß jedes Kissen nur mittels eines einzigen Doppelschlauches an das Versorgungsgerät angeschlossen werden kann,
- FIG. 3: ein Versorgungsgerät, das bei der Ausführung gemäß FIG. 1 verwendet wird,
- FIG. 4: eine Kupplungseinrichtung, die bei einem Versorgungsgerät für Kissen entsprechend FIG. 2 mit koaxialen Ein- und Auslaßöffnungen verwendet wird,
- FIG. 5 bis FIG. 7: Teile der Kupplungseinrichtung gemäß FIG. 4,
- FIG. 8: die Kupplungseinrichtung gemäß FIG. 4 im entkuppelten Zustand, wobei sich Absperrventile der Kupplungsteile in Schließstellung befinden,
- FIG. 9: die Kupplungseinrichtung gemäß FIG. 4 in einem teilgekuppelten Zustand, wobei einzelne Ventile geöffnet sind, jedoch mindestens ein Hauptventil geschlossen ist,
- FIG. 10: die Kupplungseinrichtung im vollständig gekuppelten Zustand, wobei alle Ventile offen sind,
- FIG. 11: eine Draufsicht auf den Deckel des Versorgungsgerätes mit fünf Versorgungsanschlüssen,
- FIG. 12: eine Seitenansicht des Deckels gemäß FIG. 11,
- FIG. 13: eine Längschnittansicht durch den Deckel längs der Linie 13-13 der Figur 11,
- FIG. 14: eine Längsschnittansicht durch den Deckel längs der Linie 14-14 der Figur 11,
- FIG. 15: eine Unteransicht eines an der Innenseite des Deckels befestigten Einsatzes und
- FIG. 16: eine Explosiondarstellung der am Deckel des Versorgungsgerätes befestigten Einbauteile für die Versorgungskammer.

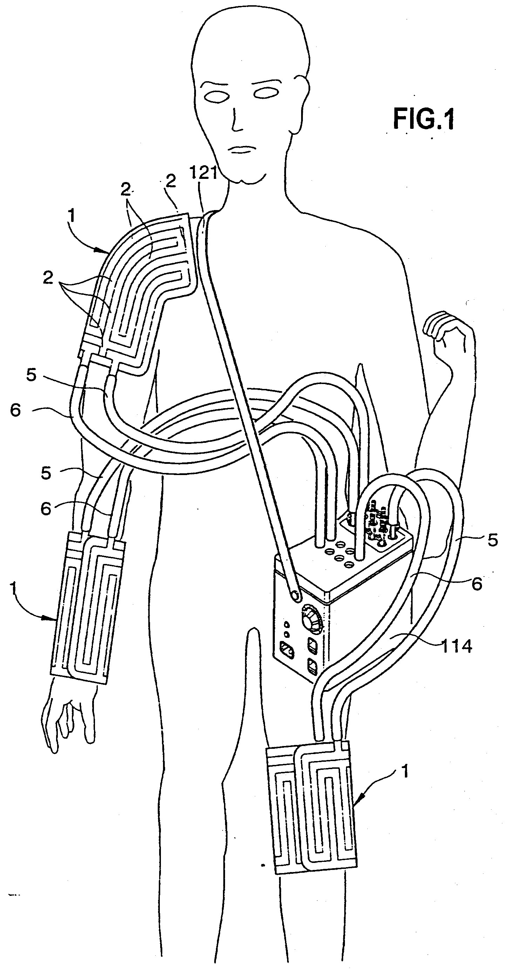

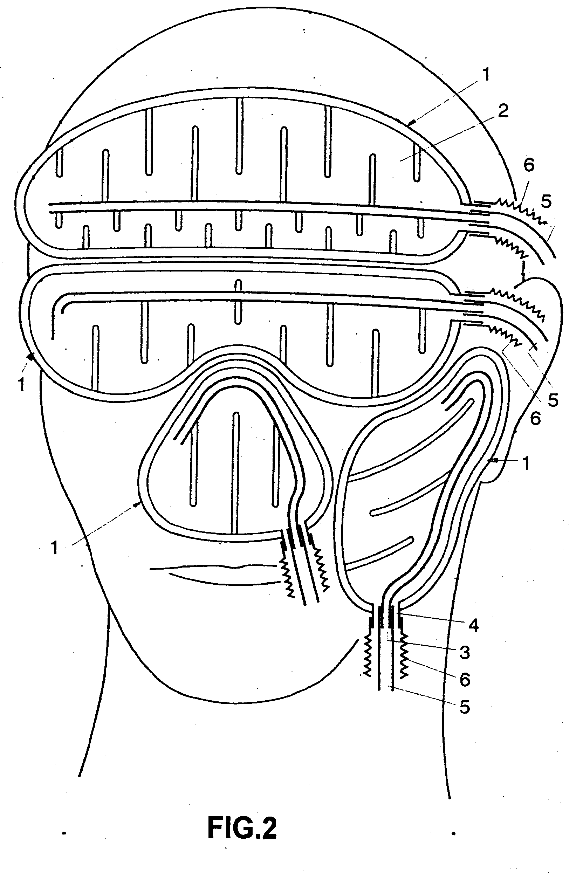

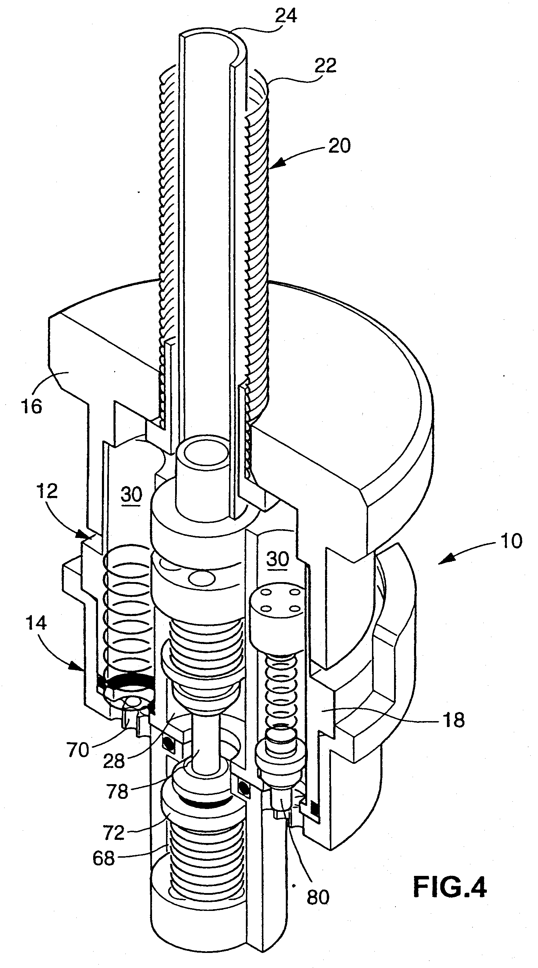

Die Einrichtung zum Temperieren von Hautpartien umfaßt mehrere Kissen 1, die jeweils ein Flüssigkeitskanalsystem 2 mit Einlaßöffnung 3 und Auslaßöffnung 4 sowie Zulaufleitung 5 und Rücklaufleitung 6 aufweisen. Gemäß FIG. 1 sind die Zulauf- und Rücklaufleitungen 5, 6 separat an ein Versorgungsgerät 114 angeschlossen. Bei der Ausführung gemäß FIG. 2 verlaufen die Zu- und Rücklaufleitungen 5, 6 koaxial und bilden zusammen einen Doppelschlauch 20 (FIG. 4).

Die Zu- und Rücklaufleitungen 5, 6 können auf entsprechende Anschlußnippel der Kissen 1 lösbar aufgesteckt oder fest mit dem Kissen verbunden sein.

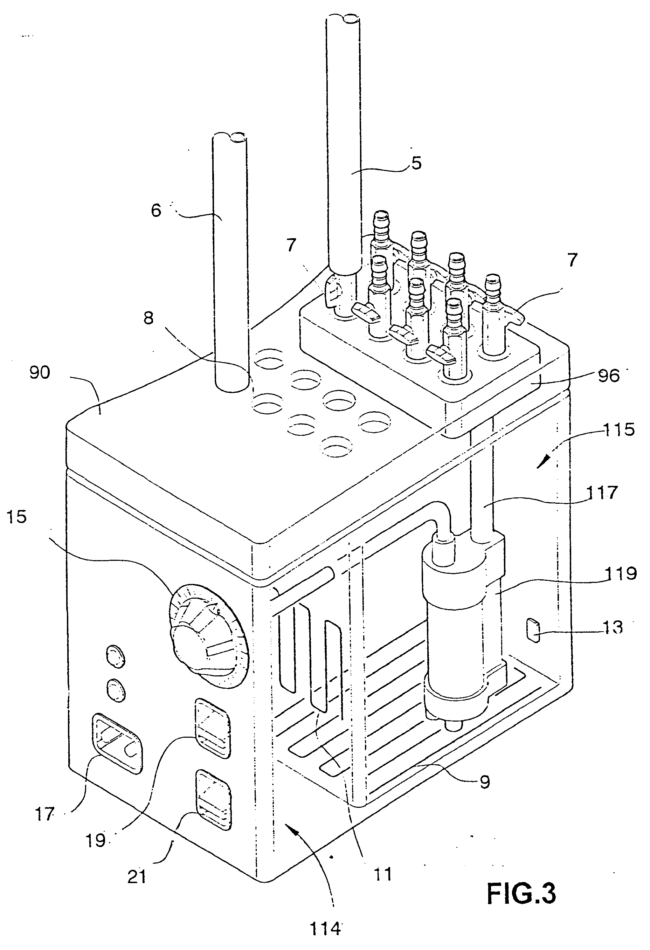

Gemäß FIG. 3 weist das Versorgungsgerät 114 einen oben offenen Behälter 115 auf, der außenseitig wärmeisoliert ist und der von einem Deckel 90 des Versorgungsgerätes abgedeckt ist. Im Umfangsbereich des Behälters 115 sind im Deckel mehrere Löcher 8 vorgesehen, durch die die Rücklaufleitungen 6 der Kissen durch den Deckel hindurchgesteckt werden können, sodaß sie direkt in den Behälter 115 entleeren können. An der Unterseite des Deckels 90 wird eine Versorgungskammer 96 gebildet, aus welcher Versorgungsanschlüsse nach oben herausragen, die mit eingebauten Absperrventilen 7 versehen sind. Die Versorgungskammer 96 ist mittels eines Verbindungsschlauches 117 mit einer elektrischen Tauchpumpe 119 verbunden, die im Behälter 115 angeordnet ist. An der Bodenseite des Behälters 115 befindet sich eine Heizschlange, die elektrisch beheizt wird. Weiterhin ist eine Kühlschlange 11 vorgesehen, die hier nur an einer Wand des Behälters 115 gezeigt ist, sich jedoch vorzugsweise an mehreren Wänden befindet. Das Versorgungsgerät 114 weist weiterhin einen Temperaturfühler 13 im Inneren des Behälters 115, einen einstellbaren Thermostat 15, einen Elektroanschluß 17 zum Laden eines im Versorgungsgerät 114 außerhalb des Behälters 115 untergebrachten Akkus sowie Schalter 19 und 21 jeweils zum Ein- und Ausschalten der Heiz- und Kühleinrichtungen auf. Außerhalb des Behälters 115 ist im Versorgungsgerät 114 ein Kälteaggregat untergebracht, das hier nicht dargestellt ist und entweder aus Thermoelementen oder einer Kompressionskälteanlage besteht.

In FIG. 3 ist ein Kissen mittels der Zu- und Rücklaufleitungen 5, 6 an das Versorgungsgerät 114 angeschlossen. Das der Zulaufleitung 5 zugeordnete Absperrventil 7 im Versorgungsanschluß ist voll geöffnet. Die anderen Absperrventile 7 der Versorgungsanschlüsse sind geschlossen. Soll ein weiteres Therapiekissen 1 an das Versorgungsgerät 114 angeschlossen werden, so ist dies möglich, ohne den Temperierbetrieb des schon angeschlossenen Kissens zu unterbrechen. Es ist lediglich nötig, die Rücklaufleitung 6 des weiteren Kissens 1 in ein Loch 8 des Deckels 90 einzustecken und die Zulaufleitung 5 auf einen freien Versorgungsanschluß aufzustecken, wonach dieser durch Betätigen des Absperrventils 7 geöffnet wird. Je nach Größe des jeweils angeschlossenen Kissens wird das Absperrventil mehr oder weniger weit geöffnet. Dank dieser Dosierfunktion der Absperrventile können die Durchflußmengen in den jeweiligen Kissen 1 individuell gewählt werden.

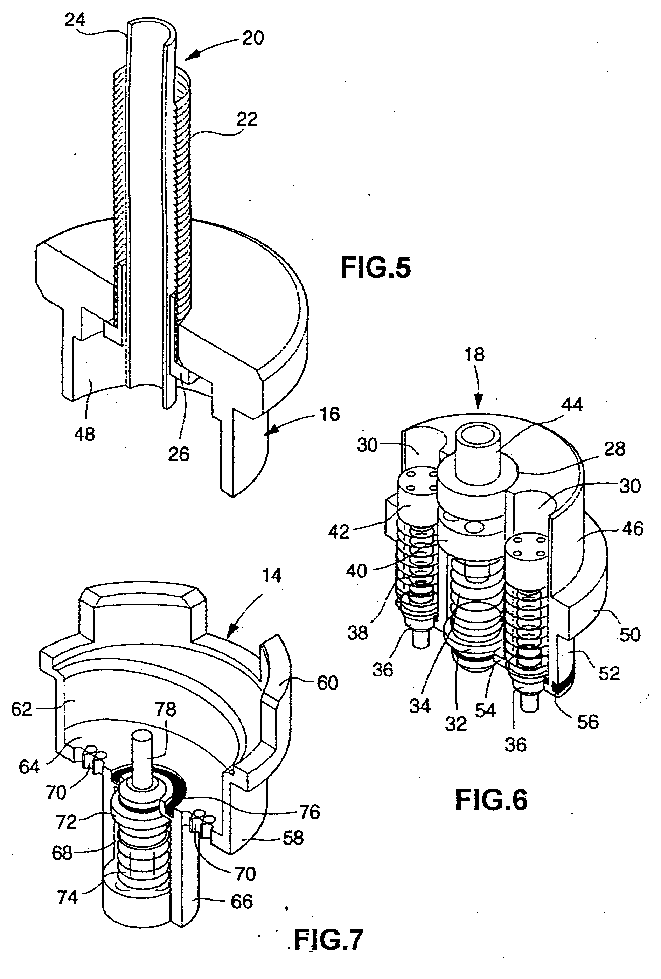

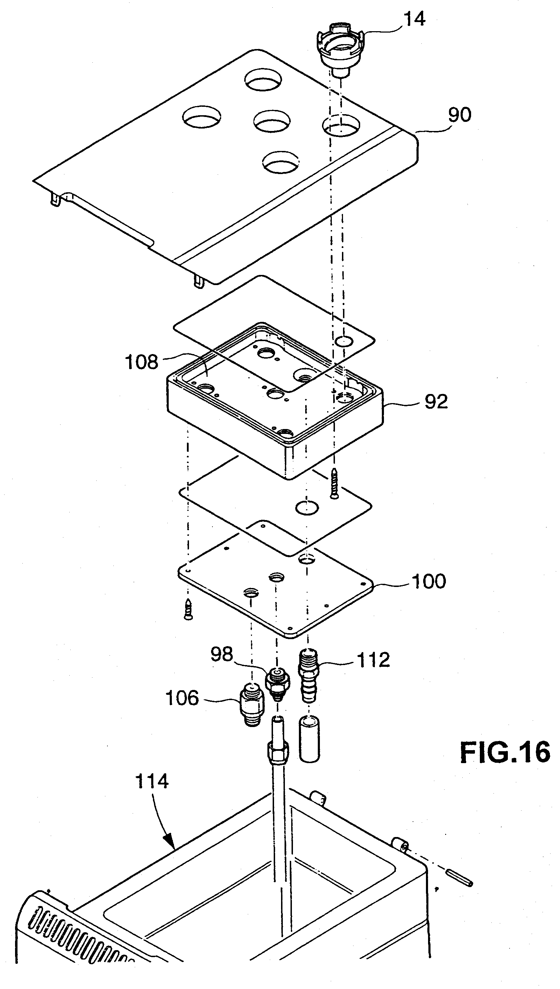

Die in den Figuren 4 - 16 veranschaulichte Kupplungseinrichtung 10 ist besonders anwenderfreundlich, da mit einem einzigen Kupplungsvorgang sowohl die Zulaufleitung 5 als auch die Rücklaufleitung 6 an das Versorgungsgerät 114 angeschlossen werden können, wobei gleichzeitig Absperrventile im Versorgungsanschluß 14 als auch in einem Kupplungsteil 12 des Doppelschlauches 20 geöffnet werden. Im entkuppelten Zustand der Kupplungseinrichtung 10 sind diese Absperrventile geschlossen, sodaß Flüssigkeit weder aus dem Versorgungsanschluß 14 noch aus dem Kupplungsteil 12 austreten kann. Diese Kupplungseinrichtung 10 ist außerordentlich sicher, verhindert ein Vertauschen der Zulauf- und Rücklaufleitungen 5, 6 und läßt sich gemäß einer Ausgestaltung arretieren, sodaß sie auch für andere Anwendungen, beispielsweise bei der Dialyse für die Hinleitung und Rückführung des Blutes von Patienten eingesetzt werden kann.

Die Kupplungseinrichtung 10 besteht aus dem Kupplungsteil 12 und dem Versorgungsanschluß 14. Das Kupplungsteil 12 hat einen Kopf 16 und einen Fuß 18. Der Kopf 16 besteht aus einer Flanschbuchse, in der ein Außenschlauch 22 eines Doppelschlauches 20 mittels einer Klemmhülse 26 abdichtend gehaltert ist. Ein Innenschlauch 24 durchsetzt die Klemmhülse 26 mit Radialabstand. Die Zuleitung 5 (FIG. 2) wird durch den Innenschlauch 24 gebildet. Die Rücklaufleitung 6 wird im Ringraum zwischen Außenschlauch 22 und Innenschlauch 24 gebildet. Der Fuß 18 hat eine koaxiale Zentralkammer 28 und zwei im Durchmesser kleinere ebenfalls zylindrische Zusatzkammern 30, die einander diametral und im gleichen Abstand von der Hauptachse gegenüber liegen. In allen drei Kammern 28, 30 sind Absperrventile angeordnet, die Ventilkörper 32 bzw. 36 und Ventilfedern 34 bzw. 38 aufweisen. Die Ventilfedern stützen sich rückseitig an, mit Lochkränzen versehenen Stützkörpern 40 bzw. 42 ab. In den Zentralkammem 28 ist oben eine Flanschbuchse 44 eingepreßt, auf die der Innenschlauch 24 paßt. Dank der Zweiteiligkeit des Kupplungsteils 12 ist eine sehr einfache Montage des Doppelschlauches 20 möglich, denn der Außenschlauch 22 wird im Kopf 16 abdichtend verklemmt und der Innenschlauch 24 wird vor der Montage der Teile etwas aus dem Außenschlauch 22 herausgezogen, was durch dessen Spiralschlauchausbildung möglich ist und auf die Flanschbuchse 44 aufgesteckt und beim Zusammenschieben von Kopf 16 und Fuß 18 im Außenschlauch 22 zentriert.

Der Fuß 18 hat eine obere kreiszylindrische äußere Umfangsfläche 46, die mit der inneren Umfangsfläche 48 des Kopfes eine Klemmsitzpassung bildet. Nach dem Zusammendrücken der beiden Teile 16, 18 ist das Kupplungsteil 12 dicht.

Etwa im Mittelbereich hat der Fuß 18 einen äußeren Ringflansch 50, an den sich nach unten eine untere Umfangsfläche 52 mit elliptischem oder ovalem Umriß anschließt. Diese Umfangsfläche 52 wird durch eine Endwand 54 geschlossen, so daß sich ein topfförmiger Aufbau ergibt. Die drei Kammern 28, 30 haben in dieser Endwand 54 Mündungsöffnungen, die als Ventilsitze für die Ventilkörper 32, 36 ausgebildet sind. Nahe dem unteren Ende der Umfangswand 52 ist in dieser ein O-Ring 56 angeordnet.

Der Versorgungsanschluß 14 ist als Kupplungsdose ausgebildet, die einen kreiszylindrischen Außenumfang 58 mit an diesen radial nach außen anschließenden und sich aufwärts erstreckenden Rastvorsprüngen 60 hat. Im Inneren des Versorgungsanschlusses 14 befindet sich eine zylindrische Ausnehmung, deren Umfangsfläche 62 zu der äußeren Umfangsfläche 52 des Fußes 18 des Kupplungsteils 12 komplementär ist. Im Ausführungsbeispiel ist diese Ausnehmung elliptisch konturiert. Diese Ausnehmung endet in einer Endwand 64, in der ein, in einem Stutzen 66 ausgebildeter Zentralkanal 68 sowie zwei radial im Abstand einander diametral gegenüber liegende zusätzliche Kanäle 70 in Form von Lochkränzen münden. Im zentralen Kanal 68 sind ein Ventilkörper 72 mit Dichtring und eine Ventilfeder 74 angeordnet, die ein Absperrventil bilden. Die Mündung des zentralen Kanals 68 in der Endplatte 64 ist von einer oben offenen Ringnut umgeben, in der ein O-Ring 76 eingesetzt ist. Vom Ventilkörper 72 erstreckt sich ein Stößel 78 koaxial nach oben in die von der Umfangsfläche 62 umschlossene Vertiefung. Die beiden Ventilkörper 36 des Kupplungsteils 12 weisen abwärts gerichtete Stößel 80 auf.

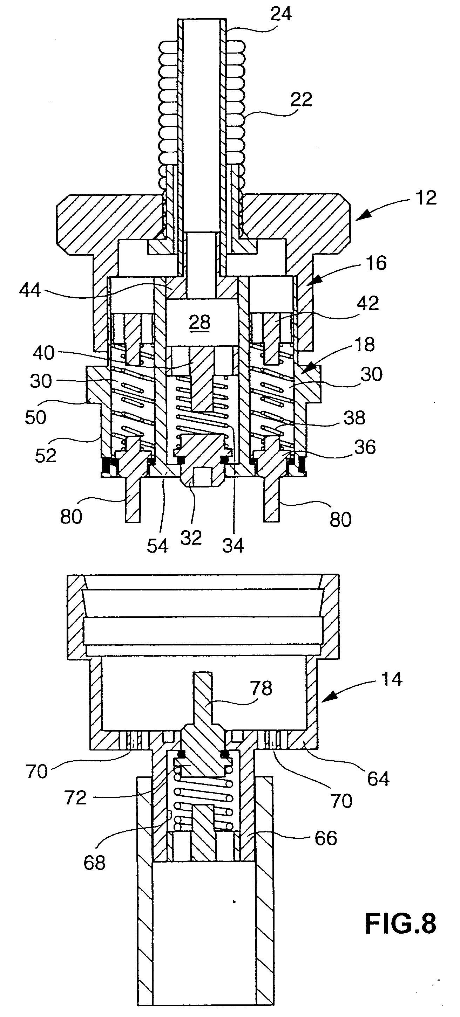

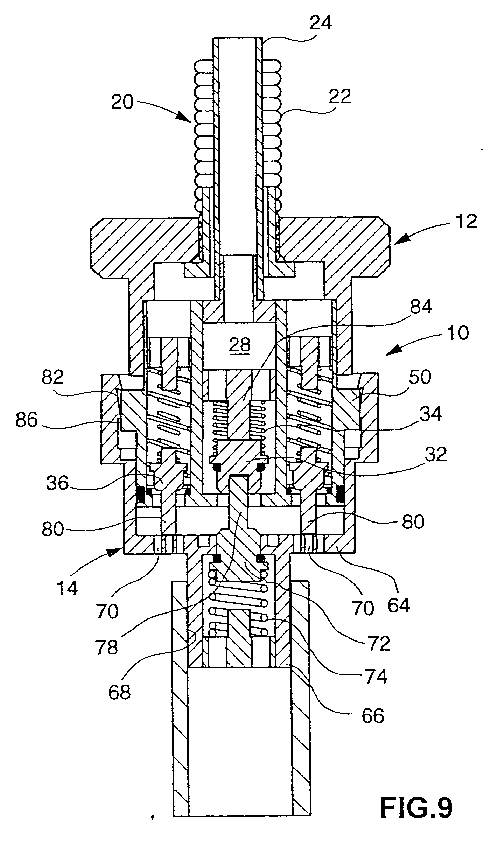

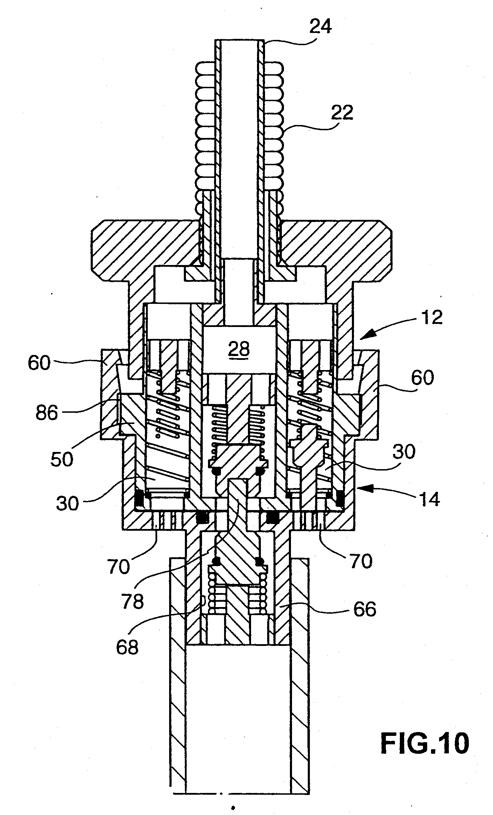

In der Entkupplungsstellung gemäß FIG. 8 sind die Absperrventile im Kupplungsteil 12 und in der Versorgungsanschluß 14 geschlossen. Wird nun das Kupplungsteil 12 in den Versorgungsanschluß eingefahren, wozu es aufgrund der elliptischen Umfangsflächen 52, 62 nur zwei definierte Winkelstellungen gibt, so dichtet zuerst der O-Ring 56 am Kupplungsteil 12 die elliptisch konturierte Kammer des Versorgungsanschlusses 14 ab. Beim weiteren Einschieben stoßen die Stößel 80 auf die Endfläche 64 und der Stößel 78 fährt in ein stirnseitiges Führungsloch des Ventilgliedes 32 ein. Ein kurzer weiterer Schiebehub des Kupplungsteils 12 hat zur Folge, daß die Absperrventile mit den Ventilgliedern 32, 36 des Kupplungsteils 12 öffnen. Diese Stellung ist in FIG. 9 gezeigt. Dabei rastet der Umfangsflansch 50 in einer ersten Raststufe 82 der hochstehenden Rastvorsprünge 60 ein. Da die Ventilfeder 74 im Zentralkanal 68 des Versorgungsanschlusses 14 stärker ausgebildet ist, als die Ventilfeder 34 in der zentralen Kammer 28 des Kupplungsteils 12, bleibt das Ventilglied 72 im zentralen Kanal 68 in Schließstellung und das Ventilglied 32 in der zentralen Kammer 28 des Kupplungsteils 12 kommt an einem Hubbegrenzungszapfen 84 zur Anlage. Flüssigkeit kann somit aus dem Innenschlauch 24 und dem Ringraum zwischen Innenschlauch 24 und Außenschlauch 22 durch die Kanäle 70 auslaufen. Durch weiteres Einschieben des Kupplungsteils 12 wird nunmehr das Ventilglied 72 vom Ventilsitz abgehoben, so daß unter Druck stehende Flüssigkeit durch den Zentralkanal 68 des Versorgungsanschlusses 14 in die zentrale Kammer 28 des Kupplungsteils 12 einströmen kann. In der Kupplungsstellung verrastet der Flansch 50 in einer zweiten Raststufe 86 an den hochstehenden Rastvorsprüngen 60. Diese Stellung ist in FIG. 10 gezeigt. Die zentrale Kammer 28 ist mit dem zentralen Kanal 68 axial in der Hauptachse der Kupplung ausgerichtet und die beiden zusätzlichen Kammern 30 im Kupplungsteil 12 sind mit den zusätzlichen Kanälen 70 ebenfalls jeweils axial ausgerichtet.

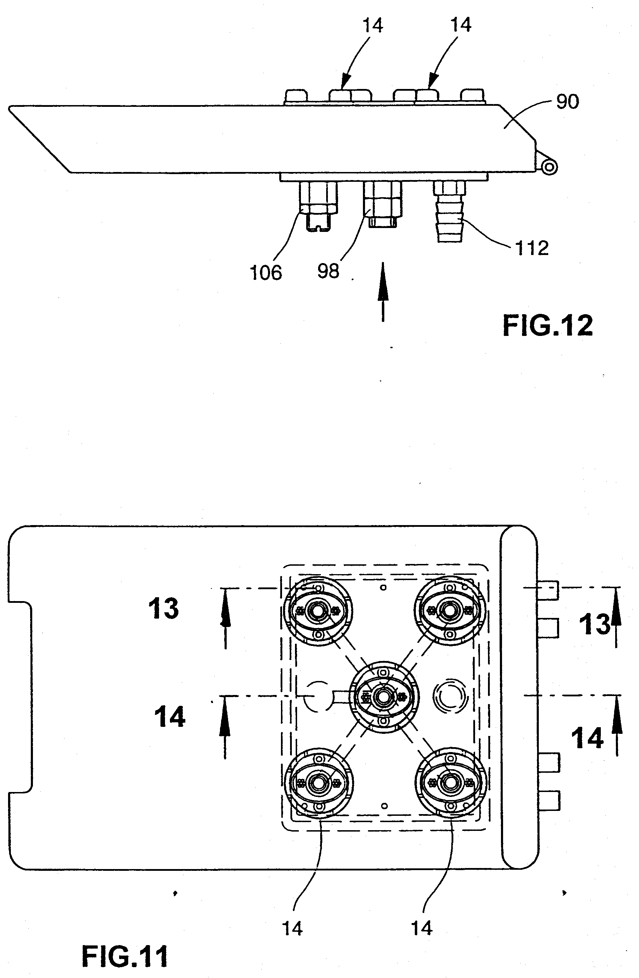

Wie die Figuren 11 bis 16 zeigen, hat das Versorgungsgerät 114 einen Deckel 90, in den von außen fünf Versorgungsanschlüsse 14 eingesetzt sind und von unten her ist an dem Deckel 90 unter Einlage einer stirnseitigen ringsum laufenden Dichtung ein Einsatz 92 befestigt, der eine Anzahl Bohrungen 94 aufweist, in die die Stutzen 66 der fünf Versorgungsanschlüssen 14 dank eingesetzter O-Ringe abdichtend eingreifen. Die 5 Bohrungen 94 stehen mit einem, eine Versorgungskammer 96 bildenden bodenseitigen Nutensystem miteinander in Verbindung, das mit einem zentralen Schlauchanschluß 98 kommuniziert, der an einer Abdeckplatte 100 angeschraubt ist, welche die Versorgungskammer 96 im Einsatz 92 schließt. Die Versorgungskammer 96 weist eine Zweignut 102 auf, die zu einer Kammer 104 führt, die mit einem Überdruckventil 106 kommuniziert, das in der Abdeckplatte 100 gehaltert ist. Der Einsatz 92 weist an der dem Deckel 90 zugewandten Seite eine großflächige, den Bereich aller fünf Versorgungsanschlüsse 14 abdeckende Tasche 108 auf, in die die Kanäle 70 der Versorgungsanschlüsse 14 frei entleeren können. In eine Entleerungsbohrung 110 des Einsatzes 92 ist ein, eine entsprechende Bohrung in der Abdeckplatte 100 durchsetzender Schlauchnippel 112 eingeschraubt.

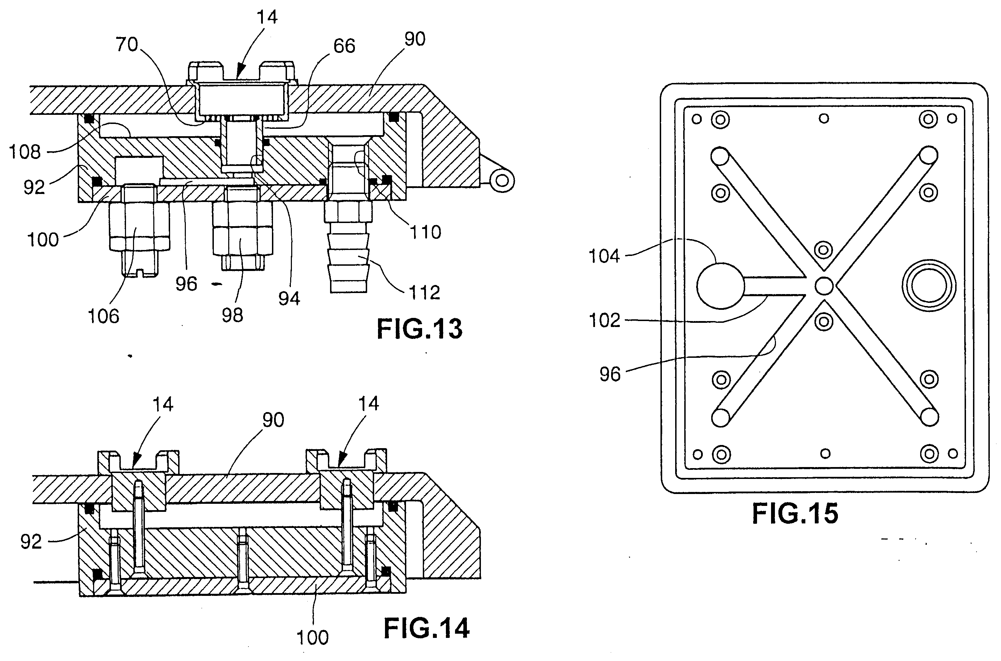

FIG. 14 veranschaulicht, daß der Einsatz 92 mit jeder der 5 Versorgungsanschlüsse 14 durch zwei lange Schrauben am Deckel 90 festgeschraubt ist und die Abdeckplatte 100 mittels sechs kürzerer Randschrauben am Einsatz 92 angeschraubt ist. Ein von der Druckseite der Pumpe 119 kommender Schlauch 117 ist mit dem zentralen Anschluß 98 verbunden und ein entsprechender Schlauch, der auf den Nippel 112 aufgesteckt ist, führt in den Behälter 115 zurück.

Die Versorgungsanschlüsse 14 Sind in den Figuren mit einfachen Auslaßkanälen 70 dargestellt, da diese in die gemeinsame Tasche 108 im Einsatz 92 entleeren können. Es versteht sich, daß der Versorgungsanschluß 14 aber auch entsprechend dem Kupplungsteil 12 zur Verbindung mit einem Doppelschlauch 20 ausgebildet sein kann. In diesem Fall weist der Versorgungsanschluß 14 ebenfalls einen Kopf 16 und einen Fuß 18 auf und statt der Kanäle 70 sind Ventilkammern 30 wie beim Kupplungsteil 12 vorgesehen. Nach dem Abziehen des Kupplungsteils 12 vom Versorgungsanschluß 14 sperren dann also auch die zusätzliche Rücklaufventile im Versorgungsanschluß, so daß aus den beiden miteinander zu verbindenden Doppelschläuchen 20 keine Flüssigkeit austreten kann.

Die erfindungsgemäße Kupplungseinrichtung ermöglicht es, daß das Kupplungsteil 12 aus dem Versorgungsanschluß 14 herausgezogen werden kann, wobei die Absperrventile selbstätig schließen und zwar bevor die Dichtung zwischen den Kupplungsteilen aufgehoben ist. Wenn sich der Versorgungsanschluß gemäß Ausführungsbeispiel im Deckel 90 des Versorgungsgerätes 114 befindet, kann die restliche Flüssigkeit aus dem Versorgungsanschluß in den Behälter 115 ausfließen, sodaß keinerlei Flüssigkeit verloren geht. Die Öffnung der Absperrventile wird nur bewirkt, wenn sich das Kupplungsteil 12 in der definierten Kupplungsstellung befindet. Fehlbedienungen sind also ausgeschlossen. Das Absperrventil im Versorgungsanschluß 14 wird in der letzten Phase der Einsteckbewegung des Kupplungsteils 12 geöffnet. Dabei wird erreicht, daß eine zweischrittige Arbeitsweise derart erfolgt, daß in der ersten Kupplungsphase die beiden Kupplungsteile 12, 14 dicht miteinander verbunden und die Absperrventile im Kupplungsteil 12 geöffnet werden, sodaß eine Entleerung der Zulauf- und Rücklaufleitungen 5, 6 einschließlich des Wasservorrates im Kissen 1 möglich ist. Im anschließenden zweiten Kupplungsschritt wird dann erst die unter Druck stehende Versorgungskammer 96 mit der Zulaufleitung 5 verbunden.

Die unrunde Ausbildung der Kupplungseinrichtung ermöglicht es in einer Abwandlung zu der in den Figuren dargestellten Ausführung, zwei gleichgroße Kammern 28, 30 in verschiedenen Hälften des Kupplungsteils 12 vorzusehen. Bei der dargestellten Ausführung mit einer Zentralkammer 28 und zwei kleineren Seitenkammern 30 im Kupplungsteil 12 ist ohne weiteres auch eine kreiszylindrische Ausbildung der Umfangsflächen von Kupplungsteil 12 und Versorgungsanschluß 14 möglich. Dabei ist eine Axialführung des Kupplungsteils 12 im Versorgungsanschluß 14, z.B. durch Axialrippen und Axialnuten vorteilhaft, sodaß eine Kupplung nur in einer oder allenfalls zwei um 180° versetzten Drehstellungen möglich ist. Am Ende der Einsteckbewegung sollte die Axialführung aufgehoben werden, sodaß das Kupplungsteil 12 in die eigentliche Kupplungsstellung um z.B. um 45° oder 90° verdreht werden kann. Dadurch wird gleichzeitig eine Verriegelung der Kupplungsteile bewirkt und sichergestellt, daß die Kupplungsstellung auch wirklich erreicht wird. Diese Drehbewegung des Kupplungsteils 12 kann auf einer schraubenförmigen Bahn mit geringer Steigung erfolgen, sodaß die Kupplungsteile mit einer ausreichend hohen axialen Dichtkraft aneinander gehalten werden.

## Patentansprüche

1. Kupplungseinrichtung zum Verbinden einer medizinischen Therapieeinrichtung, insbesondere eines Therapiekissens (1) an ein Versorgungsgerät (114), mit einem, an eine erste Leitung angeschlossenen Kupplungsteil (12) und einem, an eine zweite Leitung angeschlossenen Versorgungsanschluß (14), wobei das Kupplungsteil (12) eine geometrische Hauptachse hat, die eine Einsteckrichtung definiert und das Kupplungsteil (12) an einem Einsteckende eine zylindrische erste Umfangsfläche (52) und der Versorgungsanschluß (14) eine dazu komplementäre zweite Umfangsfläche (62) aufweist und in einer der Umfangsflächen (52, 62) ein O-Ring (56) angeordnet ist, **dadurch gekennzeichnet, daß** das Kupplungsteil (12) mindestens zwei zylindrische Kammern (28, 30) aufweist, deren Achsen parallel zur Hauptachse liegen und die in einer esten Endfläche (54) des Kupplungsteils (12) Mündungen aufweisen, daß eine der Kammern (28, 30) an die, einen Innenschlauch (24) bildende erste Leitung und die mindestens eine weitere Kammer (30) an einen, zwischen dem Innenschlauch (24) und einem Außenschlauch (22) gebildeten Ringraum einer Doppelschlauchleitung angeschlossen ist, daß der Versorgungsanschluß (14) mindestens zwei achsparallele Kanäle (68, 70) aufweist, die in einer Kupplungsstellung des Kupplungsteils (12) je mit einer der Kammern (28, 30) des Kupplungsteils (12) über gegeneinander abgedichtete Strömungswege kommunizieren, daß der Versorgungsanschluß (14) eine an die zweite Umfangsfläche (62) rechtwinklig anschließende zweite Endfläche (64) aufweist, in der die Kanäle (68, 70) jeweils Mündungen aufweisen, die in der Kupplungsposition des Kupplungsteils (12) jeweils mit den Mündungen der Kammern (28, 30) des Kupplungsteils (12) koaxial liegen, daß in einer der Endflächen (54, 64) des Kupplungsteils (12) und des Versorgungsanschluß (14) ein, die jeweilige Mündung umgebender O-Ring (76) angeordnet ist, der in der Kupplungsposition von der jeweils anderen Endfläche berührt wird, daß in mindestens einem der Kanäle (68, 70) des Versorgungsanschlußes (14) ein Absperrventil (72, 74) mit Ventilkörper (72) und einer Feder (74) angeordnet ist, die den Ventilkörper (72) in Schließstellung vorspannt, daß an einem der das Kupplungsteil (12) und den Ventilkörper /72) des Versorgungsanschlußes (14) umfassenden Bauteile ein Stößel (78) vorgesehen ist, der sich in der Kupplungsposition des Kupplungsteils (12) am jeweils anderen Bauteil abstützt und den Ventilkörper (72) in Offenstellung hält, und daß in beiden Kammern (28, 30) je ein federbetätigtes Absperrventil (32, 34, 36, 38) angeordnet ist, das einen Ventilkörper (32, 36) umfaßt, welcher in Freigabeposition des Kupplungsteils (12) die Mündung der jeweiligen Kammer (28 30) schließt, daß jedem dieser Ventilkörper (32, 36) ein parallel zur Hauptachse orientierter Hilfsstößel (80) zugeordnet ist, der in Kupplungsposition des Kupplungsteils (12) an dem Versorgungsanschluß (14) abgestützt ist, und das jeweilige Absperrventil (72, 74) der Kammer in Offenstellung hält, sodaß beim Einsetzen des Kupplungsteils (12) nacheinander zuerst Innenräume des Kupplungsteils (12) und des Versorgungsanschlußes (14) gegenüber der Umgebung abgedichtet werden, dann die Absperrventile (32, 34 und 36, 38) der Kammern (28, 30) geöffnet werden und schließlich das in dem mindestens einen, Kanal (68, 70) des Versorgungsanschlußes (14) vorgesehene Absperrventil (72, 74) geöffnet wird.

2. Kupplungseinrichtung nach Anspruch 1, **dadurch gekennzeichnet, daß** der Stößel (78) an, dem Ventilkörper (72) des in dem mindestens einen Kanal (68, 70) vorgesehenen Absperrventiles (72, 74) angeordnet ist und axial in Richtung des Ventilkörpers (32) des Absperrventils 32, 34) in einer der Kammern (28, 30) des Kupplungsteils (12) vorsteht, daß für mindestens einen dieser beiden Ventilkörper (32, 72) ein Hubbegrenzungsmittel (84) vorgesehen ist, und daß beim Kupplungsvorgang des Kupplungsteils (12) der Stößel (78) das Absperrventil (32/34) in der Kammer, (28) öffnet und den Ventilkörper (32) des Kupplungsteils (12) gegen das Hubbegrenzungsmittel (84) drückt und anschließend den Ventilkörper (72) des Versorgungsanschlußes (14) in Offenstellung bewegt, wobei die Federn (34, 74) für die beiden Ventilkörper (32, 72) unterschiedlich stark sind und die schwächere Feder (34) dem das Hubbegrenzungsmittel (84) aufweisenden Absperrventil (32, 34) zugeordnet ist.

3. Kupplungseinrichtung nach Anspruch 1, **dadurch gekennzeichnet, daß** die Umfangsflächen (52, 62) des Kupplungsteils (12) und des Versorgungsanschlußes (14) unrunde Konturen aufweisen, die ovale und eliptische Formen umfassen, und daß die beiden Kammern (28, 30) einerseits und die mit ihnen jeweils axial ausgerichteten Kanäle (68, 70) andererseits jeweils wenigstens angenähert gleich große Querschnitte aufweisen und in verschiedenen Hälften des Kupplungsteils (12) und des Versorgungsanschlußes (14) angeordnet sind.

4. Kupplungseinrichtung nach Anspruch 1, **dadurch gekennzeichnet, daß** jeweils eine Kammer (28) des Kupplungsteils (12) und ein Kanal (88) des Versorgungsanschlußes (14) zur Hauptachse koaxial liegen und das Kupplungsteil (12) mindestens zwei weitere Kammern (30) kleineren Querschnittes aufweist, deren Achsen von der Hauptachse gleich große Abstände haben und zueinander umfangsversetzt sind.

5. Kupplungseinrichtung nach Anspruch 1, **dadurch gekennzeichnet, daß** den Mündungen aller Kammern (28, 30) und aller Kanäle (68, 70) jeweils ein Absperrventil zugeordnet ist und daß die Stärken der Federn (34, 74) für die Ventilkörper (32, 72) der Absperrventile so aufeinander abgestimmt sind, daß der Kupplungsvorgang zweistufig erfolgt und in einer. Zwischenposition des Kupplungsteils, in der die einander zugewandten Endflächen (54, 64) des Kupplungsteils (12) und des Versorgungsanschlußes (14) einen Abstand haben, wenigstens eine Kammer (30) des Kupplungsteils (12) mit einem Kanal (70) des Versorgungsanschlußes (14) kommuniziert, während eine andere Kammer (28) des Kupplungsteils (12) gegenüber einem ihr zugeordneten Kanal (68) des Versorgungsanschlußes (14) abgesperrt ist.

6. Kupplungseinrichtung nach Anspruch 1, **dadurch gekennzeichnet, daß** eine Versorgungskammer (96) in einem Einsatz (92) an einer Innenseite einer, einen Deckel (90) umfassenden Außenwand des Versorgungsgerätes (114) ausgebildet ist, daß an der Versorgungskammer (96) eine Anzahl Versorgunsanschlüße (14) angeschlossen ist, daß in dem Einsatz (92) eine Anzahl paralleler Bohrungen (94) vorgesehen ist, in die je ein, an jeden Versorgungsanschluß (14) zentral angeordneter Stutzen (66) abdichtend eingreift, der jeweils einen Kanal (68) mit Absperrventil (72, 74) enthält, daß alle Bohrungen (94) durch ein, in dem Einsatz (92) ausgebildetes Kanalsystem mit einem gemeinsamen Schlauchanschluß (98) an der Innenseite des Einsatzes (94) verbunden sind, wobei das Kanalsystem die Versorgungskammer (96) bildet, daß der Einsatz (92) an einer, der Außenwand (90) zugewandten Seite eine flache Tasche (108) aufweist, die von den Stutzen (66) der Versorgungsanschlüße (14) durchsetzt werden und in die alle Kanäle (70) der Versorgungsanschlüße (14) mit Ausnahme der durch die Stutzen (66) gebildeten Kanäle (68) frei münden und daß eine in der Tasche (108) mündende Entleerungsbohrung (110) den Einsatz (92) durchsetzt.

## Claims

1. A coupling device for connecting a medical treatment device, in particular a treatment pad (1), to a supply unit (114), comprising a coupling part (12) connected to a first line and a supply connection (14) connected to a second line, wherein the coupling part (12) has a geometric main axis defining an insertion direction, and the coupling part (12) has a first, cylindrical circumferential surface (52) at one insertion end and the supply connection (14) has a complementary second circumferential surface (62), and an O-ring (56) is arranged in one of the circumferential surfaces (52, 62), characterised in that the coupling part (12) has at least two cylindrical chambers (28, 30), the axes of which extend parallel to the main axis and which have openings in a first end surface (54) of the coupling part (12), in that one of the chambers (28, 30) is connected to the first line forming an inner tube (24), and the at least one further chamber (30) is connected to an annular chamber of a dual-tube line formed between the inner tube (24) and an outer tube (22), in that the supply connection (14) has at least two axially parallel channels (68, 70) each communicating with one of the chambers (28, 30) of the coupling part (12) via mutually sealed flow paths when the coupling part (12) is in a coupling position, in that the supply connection (14) has a second end surface (64) which adjoins the second circumferential surface (62) at right angles and into which the channels (68, 70) open, the openings each being co-axial with the openings of the chambers (28, 30) of the coupling part (12) when the latter is in the coupling position, in that an O-ring (76), surrounding the respective opening, is arranged in one of the end surfaces (54, 64) of the coupling part (12) and the supply connection (14) and contacts the respective other end surface in the coupling position, in that a shut-off valve (72, 74) is arranged in at least one of the channels (68, 70) of the supply connection (14) and comprises a valve body (72) and a spring (74) biassing the valve body (72) in the closed position, in that a push rod (78) is provided on one of the components comprising the coupling part (12) and the valve body (72) of the supply connection (14) and is supported on the respective other component and holds the valve body (72) in the open position when the coupling part (12) is in the coupling position, in that a spring-operated shut-off valve (32, 34, 36, 38) is arranged in each chamber (28, 30) and comprises a valve body (32, 36) which closes the opening of the respective chamber (28, 30) when the coupling part (12) is in the release position, and in that an auxiliary push rod (80), extending parallel to the main axis, is associated with each of the valve bodies (32, 36) and is supported on the supply connection (14) and holds the respective shut-off valve (72, 74) of the chamber in the open position when the coupling part (12) is in the coupling position so that, when the coupling part (12) is inserted, inner chambers of the coupling part (12) and the supply connection (14) are first sealed relative to the environment, then the shut-off valves (32, 34 and 36, 38) of the chambers (28, 30) are opened, and finally the shut-off valve (72, 74), provided in the at least one channel (68, 70) of the supply connection (14), is opened.

2. A coupling device according to claim 1, characterised in that the push rod (78) is arranged on the valve body (72) of the shut-off valve (72, 74) provided in the at least one channel (68, 70) and projects axially in the direction of the valve body (32) of the shut-off valve (32, 34) in one of the chambers (28, 30) of the coupling part (12), in that a stroke-limiting means (84) is provided for at least one of these two valve bodies (32, 72), and in that, during the coupling process of the coupling part (12), the push rod (78) opens the shut-off valve (32, 34) in the chamber (28) and pushes the valve body (32) of the coupling part (12) towards the stroke-limiting means (84) and then moves the valve body (72) of the supply connection (14) into the open position, the springs (34, 74) for the two valve bodies (32, 72) being of different strengths and the weaker spring (34) being associated with the shut-off valve (32, 34) having the stroke-limiting means (84).

3. A coupling device according to claim 1, characterised in that the circumferential surfaces (52, 62) of the coupling part (12) and the supply connection (14) have non-round contours comprising oval and elliptical forms, and in that the two chambers (28, 30) on the one hand and the channels (68, 70) arranged co-axially therewith on the other hand each have cross-sections of at least substantially equal size and are arranged in different halves of the coupling part (12) and the supply connection (14).

4. A coupling device according to claim 1, characterised in that a chamber (28) of the coupling part (12) and a channel (88) of the supply connection (14) are arranged co-axially with the main axis, and the coupling part (12) has at least two further chambers (30) of smaller cross-section, the axes of which are at equal distances from the main axis and are circumferentially staggered.

5. A coupling device according to claim 1, characterised in that a shut-off valve is associated with each of the openings of all the chambers (28, 30) and all the channels (68, 70) and in that the strengths of the springs (34, 74) for the valve bodies (32, 72) of the shut-off valves are mutually adapted so that the coupling process takes place in two stages, and in an intermediate position of the coupling part, in which the facing end surfaces (54, 64) of the coupling part (12) and the supply connection (14) are spaced apart, at least one chamber (30) of the coupling part (12) communicates with a channel (70) of the supply connection (14), while another chamber (28) of the coupling part (12) is shut off relative to a channel (68) of the supply connection (14) associated with the chamber (28).

6. A coupling device according to claim 1, characterised in that a supply chamber (96) is formed in an insert (92) on an inner surface of an outer wall of the supply unit (114), the outer wall comprising a cover (90), in that a plurality of supply connections (14) is connected to the supply chamber (96), in that a plurality of parallel bores (94) is provided in the insert (92), a connecting piece (66), centrally arranged on each supply connection (14), engaging in each bore (94) in a sealed manner and each connecting piece (66) comprising a channel (68) with a shut-off valve (72, 74), in that all the bores (94) are connected to a common tube connection (98) on the inside of the insert (94) by means of a channel system formed in the insert (92), the channel system constituting the supply chamber (96), in that the insert (92) has a flat pocket (108) on one side facing the outer wall (90), the connecting pieces (66) of the supply connections (14) extending through the pocket (108) and all the channels (70) of the supply connections (14) opening freely into the pocket (108), with the exception of the channels (68) formed by the connecting pieces (66), and in that a discharge bore (110), opening into the pocket (108), extends through the insert (92).

## Revendications

1. Dispositif d'accouplement pour connecter un dispositif de thérapie médicale, en particulier un coussin thérapeutique (1) à un appareil d'alimentation (114), avec un élément d'accouplement (12) raccordé à un premier conduit et une connexion d'alimentation (14) raccordée à un second conduit, l'élément d'accouplement (12) ayant un axe principal géométrique définissant une direction d'engagement et l'élément d'accouplement (12) présentant, à une extrémité d'engagement, une première surface périphérique cylindrique (52) et la connexion d'alimentation (14) présentant une seconde surface périphérique (62) complémentaire à cette dernière et dans l'une des surfaces périphériques (52, 62) étant disposé un joint torique (56), caractérisé en ce que l'elément d'accouplement (12) présente au moins deux chambres cylindriques (28, 30) dont les axes sont parallèles à l'axe principal et présentant des embouchures dans une première surface d'extrémité (54) de l'élément d'accouplement (12), que l'une des chambres (28, 30) est raccordée au premier conduit formant un flexible intérieur (24) et que l'au moins une autre chambre (30) est raccordée à un espace annulaire d'un conduit, en forme de flexible double, formé entre le flexible intérieur (24) et un flexible extérieur (22), que la connexion d'alimentation (14) présente au moins deux canaux (68, 70) à axe parallèle communiquant, dans une position d'accouplement de l'élément d'accouplement (12), chacun avec l'une des chambres (28, 30) de l'élément d'accouplement (12), par l'intermédiaire de chemins de circulation rendus étanches l'un par rapport à l'autre, que la connexion d'alimentation (14) présente une seconde surface d'extrémité (64), aboutant à angle droit à la seconde périphérique (62), dans laquelle les canaux (68, 70) présentent, chacun, des embouchures qui, dans la position d'accouplement de l'élément d'accouplement (12), sont, chacune, coaxiale par rapport aux embouchures des chambres (28, 30) de l'élément d'accouplement (12), que dans l'une des surfaces d'extrémité (54, 64) de l'élément d'accouplement (12) et de la connexion d'alimentation (14) est disposé un joint torique (76), entourant l'embouchure correspondante, avec lequel, dans la position d'accouplement, vient en contact l'autre surface d'extrémité correspondante, que dans au moins l'un des canaux (68, 70) de la connexion d'alimentation (14) est disposée une soupape d'arrêt (72, 74) à corps de soupape (72) et ressort (74) prétendu, en position de fermeture, dans le corps de soupape (72), que sur l'un des éléments de construction comprenant l'élément d'accouplement (12) et le corps de soupape (72) de la connexion d'alimentation (14) est prévu un poussoir (78) qui, en position d'accouplement de l'élément d'accouplement (12), s'appuie sur l'autre élément de construction correspondant et maintient le corps de soupape (72) en position ouverte, et que dans chacune des deux chambres (28, 30) est disposée une soupape d'arrêt (32, 34, 36, 38) actionnée par ressort et comportant un corps de soupape (32, 36) fermant, en position de libération de l'élément d'accouplement (12), l'embouchure de la chambre correspondante (28, 30), qu'à chacun de ces corps de soupape (32, 36) est associé un poussoir auxiliaire (80), orienté parallèle à l'axe principal, qui, en position d'accouplement de l'élément d'accouplement (12), s'appuie sur la connexion d'alimentation (14) et maintient la soupape d'arrêt (72, 74) de la chambre en position ouverte, de sorte que lors de l'insertion de l'élément d'accouplement (12), successivement, les espaces intérieurs de l'élément d'accouplement (12), et de la connexion d'alimentation (14) sont tout d'abord obturés de manière étanche par rapport à l'environnement, ensuite les soupapes d'arrêt (32, 34 et 36, 38) des chambres (28, 30) sont ouvertes et, finalement, la soupape d'arrêt (72, 74) prévue dans l'au moins un canal (68, 70) de la connexion d'alimentation (14) est ouverte.

2. Dispositif d'accouplement suivant la revendication 1, caractérisé en ce que le poussoir (78) est disposé sur le corps de soupape (72) de la soupape d'arrêt (72, 74) prévue dans l'au moins un canal (68, 70) et fait axialement saillie en direction du corps de soupape (32) de la soupape d'arrêt (32, 34) dans l'une des chambres (28, 30) de l'élément d'accouplement (12), qu'il est prévu, pour au moins l'un de ces deux corps de soupape (32, 72), un moyen de limitation de course (84), et que, lors de l'opération d'accouplement de l'élément d'accouplement (12), le poussoir (78) ouvre la, soupape d'arrêt (32/34) dans la chambre (28) et pousse l'élément d'accouplement (12) contre le moyen de limitation de course (84) et déplace ensuite le corps de soupape (72) de la connexion d'alimentation (14) en position ouverte, les ressorts (34, 74) pour les deux corps de soupape (32, 72) étant d'épaisseur différente et le ressort le plus faible (34) étant associé à la soupape d'arrêt (32, 34) présentant le moyen de limitation de course (84).

3. Dispositif d'accouplement suivant la revendication 1, caractérisé en ce que les surfaces périphériques (52, 62) de l'élément d'accouplement (12) et de la connexion d'alimentation (14) présentent des contours non ronds, comprenant des formes ovales et elliptiques, et que les deux chambres (28, 30), d'une part, et les canaux (68, 70) alignés, chacun, axialement sur ces dernières, d'autre part, présentent, chacun, des sections au moins environ de même grandeur et sont disposés dans des moitiés différentes de l'élément d'accouplement (12) et de la connexion d'alimentation (14).

4. Dispositif d'accouplement suivant la revendication 1, caractérisé en ce que chaque fois une chambre (28) de l'élément' d'accouplement (12) et un canal (88) de la connexion d'alimentation (14) sont coaxiaux à l'axe principal et que l'élément d'accouplement (12), présente au moins deux autres chambres (30) de section inférieure dont les axes sont équidistants de l'axe principal et décalés en périphérie l'un par rapport à l'autre.

5. Dispositif d'accouplement suivant la revendication 1, caractérisé en ce qu'à chacune des embouchures de toutes les chambres (28, 30) et de tous les canaux (68, 70) est associée une soupape d'arrêt ,et que les épaisseurs des ressorts (34, 74) pour les corps de soupape (32, 74) des soupapes d'arrêt sont réglées l'une par rapport à l'autre de sorte que l'opération d'accouplement se fasse en deux étapes et, dans une position intermédiaire de l'élément d'accouplement, dans laquelle les surfaces d'extrémité (54, 64), tournées l'une vers l'autre, de l'élément d'accouplement (12) et de la connexion d'alimentation (14) sont, distantes l'une de l'autre, au moins une chambre (30) de l'élément d'accouplement (12) communique avec un canal (70) de la connexion d'alimentation (14), tandis qu'une autre chambre (28) de l'élément d'accouplement (12) est obturée par rapport à un canal (68) lui associé de la connexion d'alimentation (14).

6. Dispositif d'accouplement suivant la revendication 1, caractérisé en ce qu'une chambre d'alimentation (96) est réalisée dans un insert (92) sur une surface intérieure d'une paroi extérieure de l'appareil d'alimentation (114) comportant un couvercle (90), qu'à la chambre d'alimentation (96) sont raccordées un nombre de connexions d'alimentation (14), que dans l'insert (92) sont prévus un nombre d'alésages (94) parallèles dans chacune desquelles s'engage, de manière étanche, une tubulure (66) disposée de manière centrale sur chaque connexion d'alimentation (14) et comportant un canal (68) à soupape d'arrêt (72, 74), que tous les alésages (94) sont reliés, par un système de canaux, réalisé dans l'insert (92), à raccord de flexible commun (98), à la surface intérieure de l'insert (94), le système de canaux formant la chambre, d'alimentation (96), que l'insert (92) présente, d'un côté tourné vers la paroi extérieure (90), une poche plate (108) traversée par les tubulures (66) des connexions d'alimentation (14) et dans laquelle aboutissent librement tous les canaux (70) des connexions d'alimentation (14), à l'exception des canaux (68) formés par les tubulures (66) et qu'un alésage de vidange (110) aboutissant dans la poche (108) traverse l'insert (92).
